## Europäisches Patentamt

## European Patent Office

## Office européen des brevets

(11) Veröffentlichungsnummer: **0 068 210**
**A1**

(12) **EUROPÄISCHE PATENTANMELDUNG**

(21) Anmeldenummer: **82105064.8**

(22) Anmeldetag: **09.06.82**

(51) Int. Cl.³: **A 45 D 20/10,** A 61 N 5/08

(30) Priorität: **10.06.81 DE 3123008**

(43) Veröffentlichungstag der Anmeldung: **05.01.83**
**Patentblatt 83/1**

(84) Benannte Vertragsstaaten: **AT BE CH FR GB LI LU NL SE**

(71) Anmelder: **Kerschgens, Johann Josef,
Arabellastrasse 5/1815, D-8000 München (DE)**

(72) Erfinder: **Kerschgens, Johann Josef,
Arabellastrasse 5/1815, D-8000 München (DE)**

(74) Vertreter: **Hasse, Wilhelm Dr., Asamstrasse 8,
D-8000 München 90 (DE)**

(54) **Behandlungsgerät für Haar und Kopfhaut.**

㉝ Das Behandlungsgerät soll gleichzeitig das Haar z.B. trocknen und die Kopfhaut positiv beeinflussen durch Anregung zur besseren Durchblutung, wodurch eine Wachstumsförderung des Haares erreicht wird. Das Gerät ist auch für Räume nutzbar zu machen, indem die Luft entkeimt wird. Es besteht erfindungsgemäß aus der Kombination eines Föns (14) mit einem UV-Strahler (5), wobei sowohl Fön als auch UV-Strahler dosier- und einstellbar sind.

Johann Josef Kerschgens,
Arabellastraße 5/1815, 8000 München 81

Behandlungsgerät für Haar und Kopfhaut

Die Erfindung betrifft ein Behandlungsgerät für Haar und Kopfhaut.

Zum Behandeln von Haaren – insbesondere zum Trocknen – verwendet man einen Fön, den es in den verschiedensten Ausführungsformen gibt. Die Kopfhaut wird durch Haarwasser, Massieren usw. aktiviert, um einem Haarausfall zu begegnen.

Aufgabe der Erfindung ist es, ein Gerät zu schaffen, mit dem in "einem Arbeitsgang" sowohl die Trocknung der Haare als auch die Behandlung der Kopfhaut ermöglicht wird, ohne daß es zusätzlicher Mittel wie Haarwasser, einer Massagebehandlung oder dgl. bedarf.

Gelöst wird die gestellte Aufgabe durch die kennzeichnenden Merkmale der Ansprüche 1 bis 8, 16 und 19 sowie die der Unteransprüche.

2

Durch das erfindungsgemäße preiswerte und raumsparende Behandlungsgerät wird nicht nur das Haar getrocknet; vielmehr wird auch die Kopfhaut durch die UV-Bestrahlung behandelt, denn die UV-Strahlen werden bereits in den oberen Hautschichten absorbiert, so daß eine lokale Erwärmung eintritt. Eine so behandelte Kopfhaut aktiviert die Haarwurzeln, d.h. der Haarausfall wird verringert und unter gewissen Voraussetzungen wird der Haarwuchs sogar gefördert.

Dabei wirken die UV A fluoreszenz anregend, UV B (Dornostrahlung) rötet die Haut und wirkt antirachitisch. Es treten also spezifische biologische Wirkungen ein, nämlich Bildung von Vitamin A, Rötung und Bräunung der Haut und außerdem wird die Luft ionisiert.

Der Anwendungsbereich des erfindungsgemäßen Behandlungsgerätes erschöpft sich somit nicht nur in der Behandlung von Haar und Kopfhaut. Es kann auch zur Auffrischung von Raumluft genutzt werden, also zur Ozon-Bildung. Bezogen auf die Kopfhaut ergibt sich bei richtiger Dosierung eine Hautreizung (gegebenenfalls mit leichter Hautrötung) und eine spätere Pigmentierung. Außerdem wird Vitamin D in der Haut gebildet. Bei einer entsprechenden Behandlung wird auch die Blutbildung - was gerade in der Kopfhaut sehr wichtig ist - angeregt, gleichbedeutend mit einer allgemeinen Leistungssteigerung.

Weitere Einzelheiten der Erfindung ergeben sich aus der Zeichnung und Beschreibung, und zwar zeigt:

Fig. 1 die Ausführung eines erfindungsgemäßen Behandlungsgerätes, insbesondere in sehr schmaler Ausführung, das sich vor allem auch als Kleinstgerät gut eignet (scheibenähnliche Ausbildung),

Fig. 2 eine weitere Ausbildungsart in Stabform,

Fig. 3 einen Schnitt nach Fig. 2,

Fig. 4 einen Fön herkömmlicher Art mit einem Aufsatz, enthaltend einen UV-Strahler und

Fig. 5 eine Ansicht des Gerätes nach Fig. 4 von vorn. (Alle Darstellungen sind rein schematisch; Verkabelungen, Schalter, Startvorrichtungen für UV-Strahler und dgl. sind nicht dargestellt, weil es sich um notorisch bekannte und in jedem Fachgeschäft erhältliche Einzelteile handelt.)

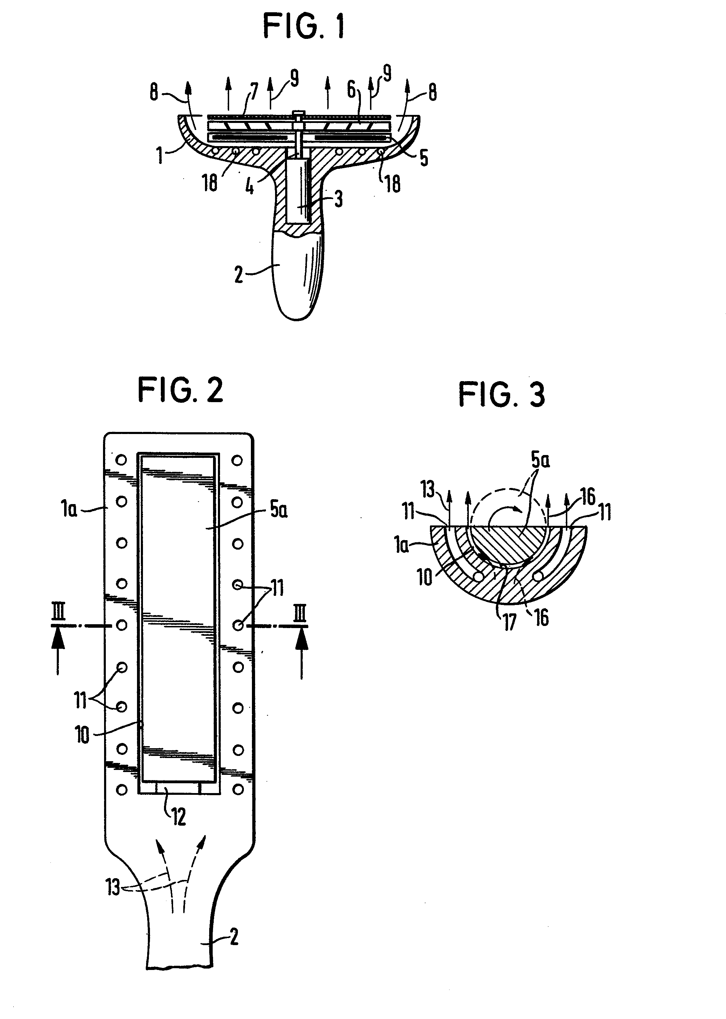

Das in Fig. 1 gezeigte Behandlungsgerät ist besonders flach (natürlich nicht zwingend) und bietet sich als Kleinstgerät (Reisegerät) an. Es besteht aus einem Gehäuse 1, in das ein elektrischer Antriebsmotor 3 (kleinste Bauart) integriert ist. Die Motor-Antriebswelle 4 ist mindestens bis zum Ventilatorrad 6 geführt und treibt dieses (gegebenenfalls abschaltbar) an. Außerdem ist ein Heizmittel 18 (z.B. Widerstandsdraht, Stab oder dgl.) dargestellt, so daß sich in Verbindung mit der Ventilatorscheibe 6 ein im Prinzip bekannter, im Aufbau jedoch völlig neuer Fön ergibt.

Der UV-Strahler 5 - hier in Ringform dargestellt - liegt zweckmäßig an der aufgezeigten Stelle, kann aber auch - in Strömungsrichtung der Pfeile 9 gesehen - vor dem Ventilatorrad 6 angeordnet sein. Nicht dargestellt sind Halterungen für den UV-Strahler 5 bzw. für das Heizmittel 18; diese können mit bekannten Schellen oder sonstigen Verbindungsmitteln am Gehäuse angeordnet werden.

Beim Normalbetrieb wird die Luft entgegengesetzt dem Pfeil 8 angesaugt und vom Ventilatorrad 6 in Richtung der Pfeile 9 geführt. Gleichzeitig kann der UV-Strahler 5 eingeschaltet werden, so daß mit der Trocknung der Haare gleichzeitig eine intensive Behandlung der Kopfhaut (s. Beschreibungseinleitung) vorgenommen wird. Zugeordnet werden kann eine Abdeckscheibe 7 (aufsetzbar und entfernbar, verdrehbar oder zusammenklappbar ähnlich einem Compurverschluß usw.), die, aus entsprechendem Material hergestellt, den UV-Strahler ganz oder teilweise abdeckt. Solch eine Scheibe kann auch perforiert sein, wobei es zweckmäßig ist, die Durchbrüche verstellbar zu gestalten, sodaß man damit eine gewünschte Dosierung erreicht.

Das Gerät kann aber auch noch anders genutzt werden, indem man beispielsweise das Heizmittel 18 ausschaltet und die Abwärme des UV-Strahlers 5 gleichzeitig zum Fönen benutzt bzw. kann man diese Abwärme auch zusätzlich zur Heizspirale nutzen.

Es kann auch zweckmäßig sein, den UV-Strahler 5 unmittelbar an der Innenfläche des Gehäuses 1 zu befestigen bzw. dort einzulagern (nicht dargestellt). Es ist auch möglich, die Anordnung auszutauschen, d.h. den UV-Strahler – in Strömungsrichtung gesehen – hinter dem Heizmittel 18 anzuordnen.

Es gehört weiter zur Erfindung, die lichte Weite des Gehäuses 1 variabel zu machen, so daß der Durchtrittsquerschnitt für die Luft, aber auch für die UV-Strahler, vergrößert oder verkleinert werden kann. Dazu könnte man Abdeckringe auf den Rand des Gehäuses 1 befestigen.

Das vorher Gesagte gilt sinngemäß für die Ausführungsform nach Fig. 2 und 3, wobei dieses Behandlungsgerät mehr länglich gestaltet ist. Im Handgriff 2 kann beispielsweise (nicht dargestellt) ein Elektromotor mit Venilator untergebracht sein. Von dort strömt entsprechend der Pfeile 13 die Heißluft beispielsweise zunächst durch einen Ringkanal und dann durch die Bohrungen 11 nach außen. Im Mittelbereich ist ein UV-Strahler 5a vorgesehen. Dieser UV-Strahler kann dort eingesteckt sein
bzw. kann er drehbar gelagert werden. Dazu bedient man
sich bekannter Führungs- und Haltemittel mit Anschlag,
um eine Drehung ganz oder teilweise zu bewirken.

Man kann auch Luft entsprechend der Pfeile 16 durch das
Gerät führen; dann müssen die Bohrungen zusätzlich oder
nur entsprechend gelegt werden. In dieser Richtung können aber auch die UV-Strahler aus dem Gerät heraustreten, wenn man beispielsweise die Innenfläche der Aufnahmeöffnung 10 mit reflektierendem Material beschichtet. Auch hier kann - im vorbeschriebenen Sinne nach
Fig. 1 - dem UV-Strahler 5a ein Abdeckelement zugeschaltet werden, das beispielsweise auf der Breitseite
19 befestigt wird (verschiebbar, klappbar usw.). Entsprechende Bohrungen sind vorgesehen, damit der Luftstrom entsprechend dem Pfeil 13 austreten kann.

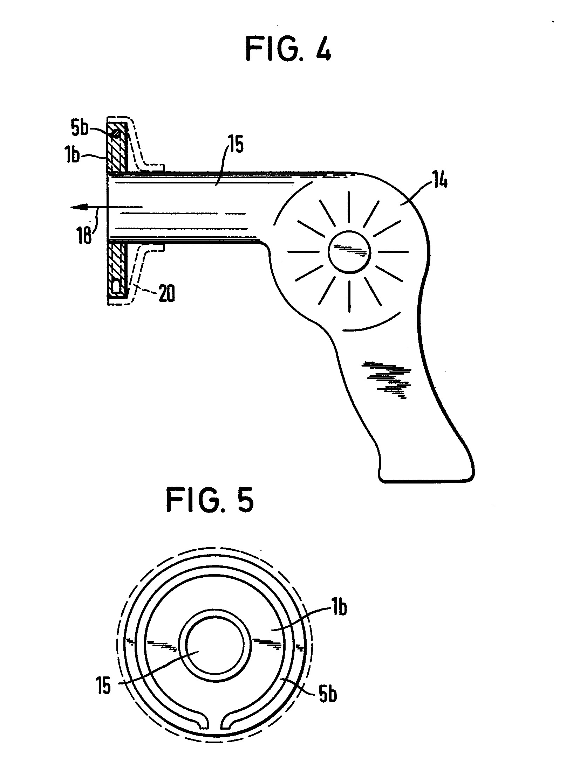

Eine sehr einfache Ausführungsform zeigt Fig. 4 in Verbindung mit Fig. 5. Hier wird ein an sich bekannter Fön
14 im Sinne der Erfindung aktiviert, d.h. man schiebt
über das Fönrohr 15 einen UV-Strahler 5b, der zweckmäßig in einem Gehäuse 1b untergebracht ist. Das Befestigen erfolgt mit bekannten Mitteln, beispielsweise
kann am Fönrohr 15 im äußeren Bereich ein Gewinde und in der Innenbohrung des Gehäuses 1b ein Gegengewinde
vorgesehen sein, so daß man den UV-Strahler 5b nur
aufschraubt. Es kann aber auch mit einer zwischengeschalteten Gummimanschette leicht entfernbar aufgeschoben werden (Klemmwirkung) bzw. sind andere Befestigungsmittel (Flansch mit Schrauben usw.) denkbar.
Auch hier werden natürlich die Verkabelungen entsprechend durchgeführt bzw. kann man auf dem Fönrohr 15
Kupplungsanschlüsse zur Zuführung des Stromes vorsehen.

Gestrichelt ist die Möglichkeit eines formschöneren
Gehäuses 20 angedeutet, das den UV-Strahler 5b aufnimmt.

Es sei noch erläutert, daß alle Merkmale, die zu den
einzelnen Ausführungsformen dargestellt sind, auch in
beliebiger Kombination bei den anderen Ausführungsformen Anwendung finden können.

Um den Luftstrom zu regulieren, können an den Ansaugöffnungen verstellbare Abdeckungen vorgesehen sein,
beispielsweise verschiebbare Abdeckbleche, so daß die
Eintrittsöffnungen (z.B. Schlitze) ganz oder teilweise
geschlossen werden können.

**PATENTANWALT**

**DR. WILHELM HASSE**

**DIPLOMINGENIEUR**

8000 MÜNCHEN , 9.6.1981/R

P Gm 4477/Hi

0068210

S t ü c k l i s t e

(Bestandteil der Anmeldung)

 1 = Gehäuse, 1a, 1b
 2 = Handgriff
 3 = Elektromotor
 4 = Antriebswelle
 5 bis 5b = UV-Strahler
 6 = Ventilatorscheibe, -rad
 7 = Abdeckscheibe
 8 = Pfeil
 9 = Pfeil
10 = Aufnahmeöffnung
11 = Bohrung
12 = Lager mit Anschlag zum Verdrehen
13 = Pfeil (Luft)
14 = Fön
15 = Fönrohr
16 = Pfeil
17 = Innenfläche
18 = Heizmittel (Stab, Spirale usw.)
19 = Breitseite
20 = angedeutetes Gehäuse

**PATENTANWALT**

**DR. WILHELM HASSE** — 1 —

**DIPLOMINGENIEUR**

8000 MÜNCHEN , 9.6.1981/R

0068210

PGm 4477/Hi

P a t e n t a n s p r ü c h e

1. Behandlungsgerät für Haar und Kopfhaut,

    gekennzeichnet durch die Kombination von – jeweils mindestens einem – Fön und UV-Strahler derart, daß Fön und UV-Strahler – dosierbar – gemeinsam oder einzeln wirken.

2. Behandlungsgerät nach Anspruch 1,

    dadurch gekennzeichnet, daß der UV-Strahler auf, um oder im Fön angeordnet ist.

3. Behandlungsgerät nach wenigstens einem der vorherigen Ansprüche,

    dadurch gekennzeichnet, daß der UV-Strahler im Luftstrom des Föns liegt.

4. Behandlungsgerät nach wenigstens einem der vorherigen Ansprüche,

    dadurch gekennzeichnet, daß der Luftstrom den UV-

    Strahler seitlich umspült.

- 2 -

5. Behandlungsgerät nach wenigstens einem der vorherigen Ansprüche,

dadurch gekennzeichnet, daß der UV-Strahler verschieb-
bzw. verdreh- und arretierbar in bezug auf den Fön ist.

6. Behandlungsgerät nach wenigstens einem der vorherigen Ansprüche,

dadurch gekennzeichnet, daß der UV-Strahler ganz oder
teilweise abdeckbar ist.

7. Behandlungsgerät nach wenigstens einem der vorherigen Ansprüche,

dadurch gekennzeichnet, daß die Abstrahlungswärme des
UV-Strahlers zum Aufheizen der Luft, die der Haartrocknung dient, genutzt ist.

8. Behandlungsgerät insbesondere nach Anspruch 1, gekennzeichnet durch ein Gehäuse (1), in dem ein
Elektromotor (3) mit Antriebswelle (4) gelagert ist,
und daß UV-Strahler (5) sowie Heizspiralen (18) (-stäbe oder dgl.) in einem z.B. von einem Ventilator
(-scheibe, -rad) (6) erzeugten Luftstrom liegen (Fig.1).

9. Behandlungsgerät nach Anspruch 8,

- 3 -

dadurch gekennzeichnet, daß das Ventilatorrad (6) dem UV-Strahler (5) - in Luftströmungsrichtung gesehen - vorgeschaltet ist.

10. Behandlungsgerät nach den Ansprüchen 8 und 9, gekennzeichnet durch eine - in Strahlungsrichtung gesehen - Abdeckscheibe (7).

11. Behandlungsgerät nach den Ansprüchen 8 bis 10, dadurch gekennzeichnet, daß die Abdeckscheibe (7) eine perforierte Platte z.B. aus Metall oder eine geschlossene oder perforierte Quarzglasplatte (z.B. bis 200 nm) oder dgl. ist.

12. Behandlungsgerät nach den Ansprüchen 8 bis 11, dadurch gekennzeichnet, daß die Abdeckscheibe (7) verdreh- und arretierbar und/oder abklapp- oder aufsteckbar ist.

13. Behandlungsgerät nach den Ansprüchen 8 bis 12, dadurch gekennzeichnet, daß die Abdeckscheibe (7) unmittelbar über dem UV-Strahler (5) vorgesehen ist.

14. Behandlungsgerät nach wenigstens einem der vorherigen Ansprüche,

- 4 -

dadurch gekennzeichnet, daß der Elektromotor (3)
selber mindestens ein Teil des Gehäuses (1) ist.

15. Behandlungsgerät nach wenigstens einem der vorherigen Ansprüche,

dadurch gekennzeichnet, daß der Durchmesser des
Gehäuses veränderbar ist, z.B. durch Flexibilität
des freien Randbereiches, aufsetz- oder umklappbare
Abdeckringe, compurverschlußähnliche Randausbildung
oder dgl.

16. Behandlungsgerät insbesondere nach den Ansprüchen
1 bis 7,

dadurch gekennzeichnet, daß der UV-Strahler (5a)
in einem Gehäuse (1a) wenigstens teildrehbar gelagert und/oder umsteckbar (in verschiedene Strahlungsrichtungen) ist (Fig. 2).

17. Behandlungsgerät nach Anspruch 16,

gekennzeichnet durch ein Gehäuse (1a) mit eingebauter Föneinrichtung (Gebläse mit Heizelementen usw.),
wobei Luftaustrittsöffnungen auf der Breitseite (19)
vorgesehen sind.

18. Behandlungsgerät nach den Ansprüchen 16 und 17,

gekennzeichnet durch eine, durch eine Ausnehmung gebildete Innenfläche (17), in die paßgerecht - im Abstand - mindestens ein UV-Strahler angeordnet ist (bzw. daß die Ausnehmung der Form des UV-Strahlers angeglichen ist).

19. Behandlungsgerät insbesondere nach den Ansprüchen 1 bis 7,

gekennzeichnet durch ein einen UV-Strahler (5b) aufnehmendes Gehäuse (1b) als Aufsatz auf einen herkömmlichen Fön (14) (Fig. 4).

20. Behandlungsgerät nach Anspruch 19,

dadurch gekennzeichnet, daß das Gehäuse (1b) eine verstellbare Öffnung (z.B. Dreh-, Klemm-Verschluß) aufweist zum Festklemmen auf dem Fönrohr (15) bzw. Festsetzmittel (Schrauben) bzw. aus elastischem, selbstklemmenden Material (Gummi, Kunststoff usw.) besteht.

21. Behandlungsgerät nach wenigstens einem der vorherigen Ansprüche,

gekennzeichnet durch Reflektoren bekannter Art, die ganz oder teilweise die Innenfläche z.B. der Gehäuse (1 bis 1b) auskleiden.

- 5a -

22. Behandlungsgerät insbesondere nach Anspruch 1, 8, 16
und 19,

dadurch gekennzeichnet, daß auf der Luftansaugseite
- oder im Verlauf der Luftwege - Mittel zum ganz
oder teilweise Verschließen einiger oder aller Luftzufuhrwege vorgesehen sind.

23. Behandlungsgerät nach Anspruch 22,

dadurch gekennzeichnet, daß die Mittel aus Schiebern,
Scheiben, compurähnlichen Verschlüssen oder dgl. bestehen.

1/2

# FIG. 1

# FIG. 2

# FIG. 3

FIG. 4

FIG. 5

**Europäisches Patentamt**

**EUROPÄISCHER RECHERCHENBERICHT**

0068210
Nummer der Anmeldung

EP 82 10 5064

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | DE-B-1 042 147 (GÜRKE)<br><br>* Spalte 2, Zeile 35 - Spalte 4, Zeile 18; Abbildungen 1,2 *<br>--- | 1-5,7-9,14,22,23 | A 45 D 20/10<br>A 61 N 5/08 |
| X | DE-A-2 831 620 (JACOBI)<br>* das ganze Dokument *<br>--- | 1 | |
| A | BE-A- 860 402 (ARC-EN-CIEL COIFFURE)<br>* Patentanspüche 1,7 *<br>--- | 1 | |
| A | DE-C- 939 646 (GÜNTHER)<br><br>* Seite 2, Zeilen 23-46; Abbildung 1 *<br>--- | 2,4,8,9,17 | |
| | | | RECHERCHIERTE SACHGEBIETE (Int. Cl. ³) |
| A | DE-U-7 934 988 (BOSCH-SIEMENS)<br>* Anspruch 1 *<br>--- | 2,4,17 | A 45 D<br>A 61 N |
| A | FR-A-1 139 096 (REVEILLANT)<br><br>* das ganze Dokument *<br>--- | 2,3,7,14 | |
| A | FR-A-1 291 888 (HERAEUS QUARTZSCHAELZE)<br>* Seite 2, linke Spalte, Zeilen 15-18; Abbildung 1 *<br>---<br>-/- | 6 | |

Der vorliegende Recherchenbericht wurde für alle Patentansprüche erstellt.

| Recherchenort | Abschlußdatum der Recherche | Prüfer |
|---|---|---|
| DEN HAAG | 31-08-1982 | SIGWALT C. |

Europäisches
Patentamt

**EUROPÄISCHER RECHERCHENBERICHT**

0068210

Nummer der Anmeldung

506 1

EP 82 10 5064

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| **EINSCHLÄGIGE DOKUMENTE** | | | **Seite 2** |
| A | US-A-2 469 412 (ROEBKEN) <br><br> * Abbildungen 1,3 * <br><br> --- | 10,11, 21 | |
| A | FR-A-1 266 232 (LAURENT) <br> * das ganze Dokument * <br><br> ----- | 22,23 | |
| | | | **RECHERCHIERTE SACHGEBIETE (Int. Cl. ³)** |

Der vorliegende Recherchenbericht wurde für alle Patentansprüche erstellt.

| Recherchenort | Abschlußdatum der Recherche | Prüfer |
|---|---|---|
| DEN HAAG | 31-08-1982 | SIGWALT C. |